# FASCICULE DE BREVET EUROPEEN

(11) **EP 1 036 313 B1**
(45) Date de publication et mention de la délivrance du brevet: **19.03.2003**
(21) Numéro de dépôt: 98958320.8
(22) Date de dépôt: 03.12.1998
(51) Int. Cl.: G01N 25/12, G01N 25/02

(54) **PROCEDE ET DISPOSITIF POUR L'ETUDE DE L'EFFET D'UN FLUIDE SUPERCRITIQUE SUR LA TRANSITION D'UN MATERIAU DE L'UNE A L'AUTRE DE DEUX PHASES CONDENSEES ET LEUR APPLICATION AU CAS D'UN MATERIAU POLYMERE**
VERFAHREN UND VORRICHTUNG ZUR UNTERSUCHUNG DER AUSWIRKUNG EINER SUPERKRITISCHEN FLÜSSIGKEIT AUF DEN ÜBERGANG DER EINEN IN DIE ANDERE VON ZWEI KONDENSIERTEN PHASEN EINES MATERIALS UND DEREN ANWENDUNG FÜR EIN POLYMER
METHOD AND DEVICE FOR STUDYING THE EFFECT OF A SUPERCRITICAL FLUID ON THE TRANSITION OF A MATERIAL FROM ONE CONDENSED PHASE TO THE OTHER AND THEIR APPLICATION IN THE CASE OF A POLYMER MATERIAL

(30) Priorité: 03.12.1997 FR 9715221
(43) Date de publication de la demande: 20.09.2000
(73) Titulaire: CENTRE NATIONAL DE LA RECHERCHE SCIENTIFIQUE (CNRS), 75794 Paris Cedex 16 (FR)
(72) Inventeur: RANDZIO, Stanislaw, L., PL-04-404 Varsovie (PL); GROLIER, Jean-Pierre, E., F-63000 Clermont-Ferrand (FR)
(74) Mandataire: Schrimpf, Robert
(86) Numéro de dépôt international: FR9802612
(87) Numéro de publication internationale: WO99028731

(56) Documents cités:
- EP-A- 0 357 176
- EP-A- 0 610 953
- DD-A- 116 504
- FR-A- 2 679 650

## Description

La présente invention concerne un procédé et un dispositif pour l'étude de l'effet d'un fluide supercritique sur la transition d'un matériau de l'une à l'autre de deux phases condensées et leur application au cas d'un matériau polymère.

Les fluides supercritiques, qui ne sont ni des gaz ni des liquides et qui peuvent être comprimés progressivement d'une basse densité à une densité élevée, présentent une importance croissante comme solvants et milieux réactionnels, notamment dans l'industrie chimique, dans l'industrie pharmaceutique et dans l'industrie alimentaire.

Un exemple d'application de ces fluides dans l'industrie chimique est notamment celui des polymères, dont les fluides supercritiques permettent de contrôler le poids moléculaire et la morphologie, aboutissant à des produits modifiés nouveaux.

De telles modifications sont souvent réalisées par absorption de gaz comprimés ou par recristallisation à partir de solutions préparées avec différents solvants liquides.

Dans le cas des modifications avec absorption de gaz la substance semi-cristalline devient plus plastique et, par exemple, sa transition vitreuse peut être abaissée de plusieurs dizaines de degrés. Le défaut principal d'une telle technique est que cette propriété est pratiquement observée seulement quand la substance modifiée se trouve en présence de gaz comprimés. Cela limite beaucoup l'utilisation de cette technique.

Dans le cas de modifications par recristallisation à partir d'une solution liquide, la morphologie de la phase condensée peut être changée, mais il est bien souvent difficile de trouver des solvants liquides appropriés. Cette technique est donc pratiquement limitée aux systèmes pour lesquels les solubilités sont bien connues. En plus, la phase condensée modifiée doit être convenablement séchée et cela est un désavantage, surtout du point de vue énergétique.

Si la littérature scientifique rapporte aussi des expérimentations diverses impliquant un fluide supercritique sur des matériaux particuliers dans des conditions spécifiques, il apparaît par contre qu'il n'existe pas jusqu'à présent de dispositif permettant l'étude complète de l'effet d'un fluide supercritique sur la transition d'un matériau quelconque de l'une à l'autre de deux phases condensées, par la maîtrise de tous les paramètres significatifs.

La présente invention a pour objet un procédé et un dispositif permettant notamment l'étude de la modification de la morphologie d'une substance uniquement par fusion et recristallisation de la substance ou simplement par sa solidification à partir de l'état fluide ou liquide sous la pression (à saturation) d'un fluide supercritique dans des conditions isothermes ou isobares bien définies.

De façon typique, la gamme de pression peut aller au moins jusqu'à 400 MPa et la gamme de température peut couvrir au moins l'intervalle 220-570K.

Le dispositif qui permet l'étude de l'effet d'un fluide supercritique sur la transition d'un matériau de l'une à l'autre des deux phases condensées, comprend selon l'invention :
- une cellule (1) apte à contenir un échantillon du matériau à étudier et à supporter les pressions et températures mises en jeu dans l'étude ;
- une source de fluide supercritique et une canalisation reliant cette source à l'intérieur de la cellule pour l'introduction contrôlée de ce fluide dans la cellule en sorte qu'il soit au contact de l'échantillon de commande ;
- des moyens pour faire varier, de façon continue ou par paliers, selon un programme déterminé, la valeur d'un premier paramètre choisi par la pression (P) du fluide supercritique, la température (T) de la cellule et le volume (V) de l'échantillon dans la cellule, tout en maintenant un deuxième desdits paramètres à une valeur choisie, de façon à induire la transition d'enregistrement ;
- des moyens pour enregistrer la variation au premier paramètre, la variation du troisième paramètre et les variations (ΔH) du flux thermique dans la cellule ;
- des moyens pour effectuer des enregistrements analogues dans des conditions similaires avec un fluide neutre au lieu du fluide supercritique.

Dans des modes de réalisations préférés, le dispositif de l'invention présente encore une ou plusieurs des caractéristiques suivantes :
- la cellule contient une ampoule ouverte qui reçoit l'échantillon
- l'ampoule est à paroi souple
- le dispositif comporte une pompe haute pression dont le piston est actionné par un moteur pas à pas, des moyens pour transmettre la pression exercée par cette pompe au fluide supercritique et des moyens pour commander le moteur pas à pas et pour enregistrer les variations du nombre de pas du moteur.
- un conduit relie la sortie de pression de la pompe à ladite canalisation et contient le fluide neutre en sorte que la pression de la pompe soit transmise au fluide supercritique par le fluide neutre,
- ledit conduit comporte à la sortie de la pompe une partie de conduit située en amont du fluide neutre contenu dans le conduit et qui contient un fluide hydraulique.
- ledit conduit aboutit également dans une autre cellule identique à ladite cellule en sorte que le liquide neutre soit introduit dans cette cellule.
- la cellule ou chaque cellule est placée dans un détecteur calorimétrique entouré d'un thermostat.
- une centrale de commande et d'enregistrement est reliée au thermostat, au détecteur calorimétrique, au moteur pas à pas et à un capteur de pression qui reçoit la pression exercée par la pompe.

L'invention a également pour objet un procédé pour l'étude de l'effet d'un fluide supercritique sur la transition d'un matériau de l'une à l'autre de deux phases condensées, dans lequel :
- on introduit un échantillon du matériau et le fluide supercritique dans une cellule en sorte que le fluide soit au contact de l'échantillon ;
- on induit la transition en faisant varier, de façon continue ou par paliers, selon un programme déterminé, la valeur d'un premier paramètre choisi par la pression (P) du fluide, la température (T) de la cellule et le volume (V) de l'échantillon dans la cellule, tout en maintenant un deuxième desdits paramètres à une valeur choisie;
- on enregistre la variation du premier paramètre, la variation du troisième paramètre et la variation du flux thermique dans la cellule ;
- on recommence les opérations précédentes avec le même paramètre programmé et la même valeur choisie mais en utilisant un fluide neutre à la place du fluide supercritique, en mettant ou non le fluide neutre au contact de l'échantillon selon le cas ;
- et on compare les résultats obtenus avec les deux fluides pour évaluer l'effet du fluide supercritique sur les conditions de la transition.

Dans des modes de réalisation préférés, le procédé de l'invention présente encore une ou plusieurs des caractéristiques suivantes:
- on utilise un fluide supercritique choisi parmi le gaz carbonique, l'azote, le méthane, l'éthane, le propane ou leurs mélanges ou tout autre fluide susceptible d'être amené à l'état supercritique.
- on utilise le mercure comme fluide neutre.
- on transmet au fluide supercritique la pression d'un fluide hydraulique actionné par un piston.
- on réalise cette transmission au moyen du fluide neutre.
- on utilise ledit fluide hydraulique pour pousser le fluide neutre jusque dans la cellule.
- on déplace ledit piston par l'action d'un moteur pas à pas pour créer ladite pression et on compte le nombre de pas du moteur nécessaire pour maintenir la pression à une valeur choisie de façon à déterminer la variation du volume de l'échantillon dans la cellule pendant la transition.
- on réalise les étapes suivantes : a) introduction du fluide supercritique dans la cellule expérimentale sous la pression nécessaire pour faire travailler une pompe haute pression ; b) compression du fluide supercritique par la pompe haute pression jusqu'à la pression désirée ; c) initiation isobare de la fusion du matériau par augmentation contrôlée de la température et enregistrement simultané des changements du volume et du flux de chaleur ; d) initiation isobare de la cristallisation isobare du matériau par abaissement contrôlé de la température et enregistrement simultané des changements du volume et du flux de chaleur.
- on réalise les étapes suivantes: a) introduction du fluide supercritique dans la cellule expérimentale contenant le matériau sous la pression nécessaire pour faire travailler la pompe haute pression; b) compression du fluide supercritique par la pompe haute pression jusqu'à la pression désirée, c) initiation isotherme de la fusion de la substance par abaissement contrôlé de la pression et enregistrement simultané des changements du volume et du flux de chaleur; d) initiation isotherme de la cristallisation isotherme du matériau par augmentation contrôlée de la pression et enregistrement simultané des changements du volume et du flux de chaleur.
- on modifie ledit matériau se trouvant à l'état fluide ou liquide en abaissant sa température sous la pression contrôlée du fluide supercritique.

Parmi les applications de l'invention, on citera notamment l'étude des matériaux polymères, en particulier pour déterminer quand la solution polymère/fluide supercritique est dans un état d'équilibre et quand cette solution devient saturée.

Dans des réalisations particulières :
- on induit la transition du polymère en faisant varier la température à pression constante.
- dans les mêmes conditions de pression, on réalise un échauffement lent pour obtenir un système homogène polymère/gaz supercritique saturé puis un refroidissement lent pour faire condenser une phase micro-mousse ou nano-mousse, tout en enregistrant tous les paramètres (P,T, ΔH et ΔV) des transitions observées.
- on induit la transition du polymère en faisant varier la pression à température constante.
- dans les mêmes conditions de pression, on réalise un échauffement lent pour obtenir un système homogène polymère /gaz supercritique saturé puis une compression lente pour faire condenser une phase micro-mousse ou nano-mousse, tout en enregistrant tous les paramètres (P,T, ΔH et ΔV) des transitions observées.
- la forme stable du thermogramme, qui ne change pas au cours des transitions successives sous l'atmosphère du gaz supercritique, est l'indicateur de la fin de la formation de la nouvelle phase micro-mousse ou nano-mousse.

La description qui va suivre en regard du dessin annexé, donné à titre d'exemple non limitatif, fera bien comprendre comment l'invention peut être réalisée, les particularités qui ressortent tant du texte que du dessin faisant, bien entendu, partie de ladite invention.
- La figure 1 montre le schéma d'un dispositif pour les modifications et l'étude des propriétés de phases condensées ;
- les figures 2 et 3 sont des thermogrammes calorimétriques enregistrés dans le cas d'un polyéthylène moyenne densité traité avec du méthane supercritique ou traité avec un fluide neutre, respectivement sous des pressions de méthane de 50 et de 100 MPa ;
- la figure 4 montre la variation de la capacité thermique en fonction de la température, à pression ambiante, respectivement pour un échantillon de polyéthylène moyenne densité traité et un échantillon non traité, et
- la figure 5 est un schéma d'une variante du dispositif de l'invention.

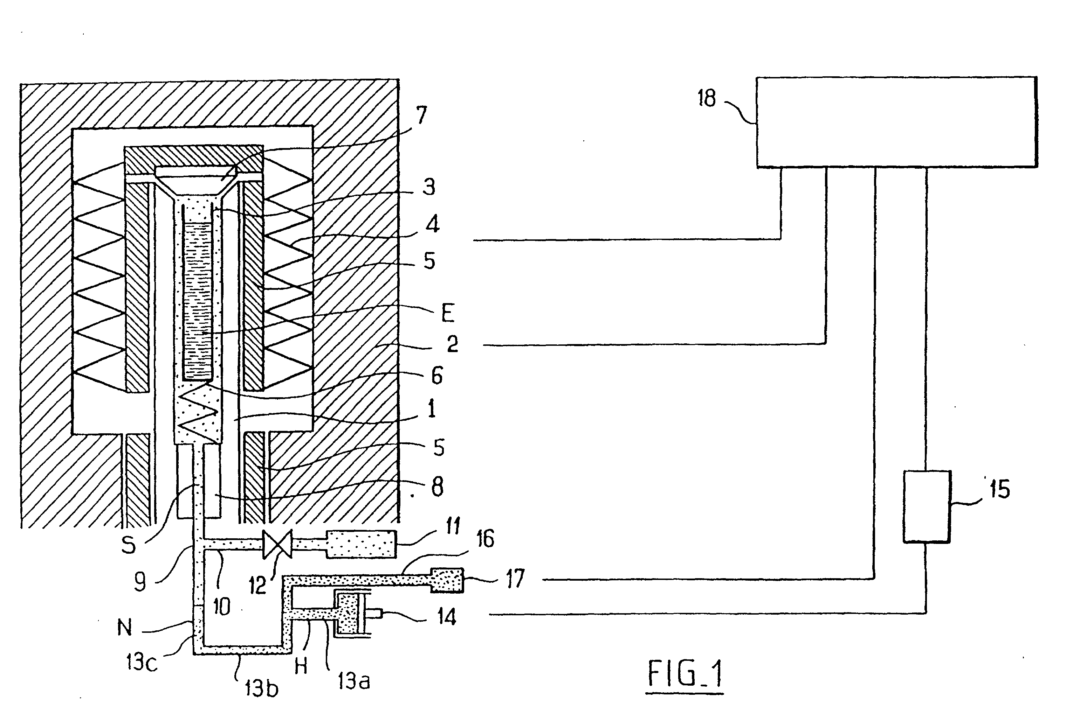

La figure 1 est un schéma d'un dispositif selon l'invention sur lequel on a représenté une cellule 1 constituée d'un tube en acier capable de résister à des pressions et à des températures élevées, placée dans un thermostat 2 et qui contient une ampoule 3 en verre, en acier ou en tout autre matériau adéquat apte à recevoir l'échantillon E à étudier. Entre le thermostat 2 et la cellule 1 sont placés un détecteur calorimétrique 4 et un échangeur de chaleur 5.

L'ampoule 3 est ouverte en partie haute et repose dans la cellule sur un ressort 6. La cellule 1 est fermée en partie haute par un bouchon 7 et communique en partie basse par un raccord 8 avec une canalisation 9 constituée par un tube capillaire en acier.

La canalisation 9 est raccordée d'une part par une branche 10 avec une source 11 de fluide supercritique sous contrôle d'une vanne 12, et, d'autre part, par une branche 13a, 13b, 13c située en amont de la jonction des canalisations 9 et 10 à la sortie d'une pompe 14 de type seringue dont le piston est commandé par un moteur pas à pas 15.

Le fluide supercritique S contenu dans la canalisation 9 reçoit la pression de la pompe 14 par l'intermédiaire d'un fluide neutre N contenue dans la canalisation 13b, 13c et d'un fluide hydraulique H contenu dans la canalisation 13a et actionné par le piston de la pompe.

Une canalisation 16 transmet la pression du fluide hydraulique à un capteur de pression 17.

Un contrôleur logique 18, relié au détecteur calorimétrique 4, au thermostat 2, au moteur pas à pas 15 et au capteur de pression 17, gère le fonctionnement du dispositif en fonction du programme que l'opérateur s'est fixé et fournit les enregistrements requis.

Ce contrôleur contrôle la pression et la température du thermostat, il enregistre le flux de chaleur par l'intermédiaire du détecteur calorimétrique 4 et il enregistre les changements du volume en comptant le nombre de pas du moteur 15.

Ce dispositif permet de faire varier n'importe lequel des trois paramètres P, V, T, par paliers ou en continu l'un des deux autres paramètres, étant maintenu constant, les changements de flux thermique dus à la variation du paramètre variable et la variation du troisième paramètre non maintenu constant étant enregistrés.

Quand on veut effectuer des mesures avec le fluide neutre, on interrompt l'arrivée du fluide supercritique et on introduit le liquide neutre jusque dans la cellule pour chasser le fluide supercritique subsistant et le remplacer par le fluide neutre.

### EXEMPLE :

On introduit dans l'ampoule un échantillon de polyéthylène de densité moyenne (MDPE, densité 938 kg/m³)

On lave l'ampoule placée dans la cellule avec du méthane supercritique (SCM) pendant quelques minutes et on ferme la cellule. On comprime le SCM sur le polymère à une pression initiale de 25-30 MPa.

On enregistre à pression constante la variation du flux thermique en fonction de la température.

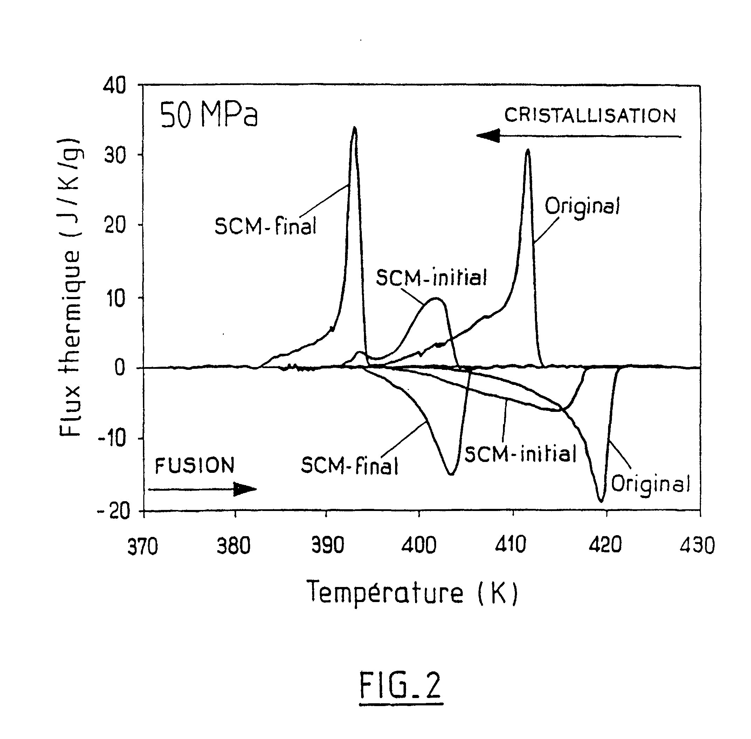

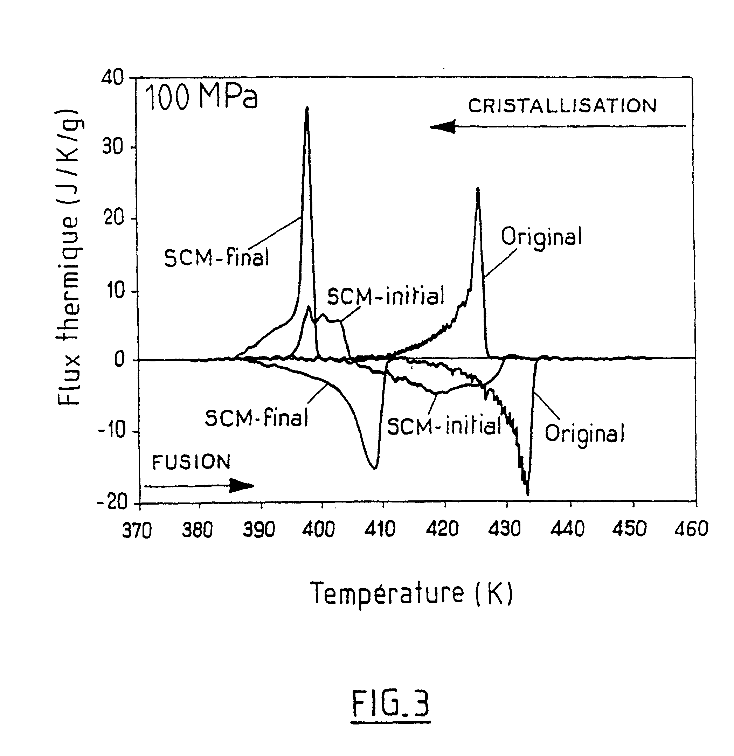

Les figures 2 et 3 montrent les thermogrammes ainsi obtenus, respectivement à une pression de 50 MPa et à une pression de 100 MPa, en présence de SCM ou en remplaçant le SCM par du mercure. Le mercure n'affectant pas le polyéthylène, les thermogrammes correspondants sont pris comme références (« original »).

Les courbes « SCM-initial » ont été obtenues durant le premier chauffage et refroidissement d'un échantillon vierge sous SCM comprimé. Les courbes « SCM-final » ont été obtenues après que l'échantillon a été fondu et recristallisé plusieurs fois sous SCM comprimé.

La comparaison de ces thermogrammes (formes et grandeurs) avec les thermogrammes originaux montrent que l'interaction du polyéthylène avec le fluide supercritique a un effet sur la fusion ainsi que sur la cristallisation.

On répète les fusions et les cristallisations jusqu'à ce que les thermogrammes ne changent plus. On constate que la texture de l'échantillon est devenue similaire à celle du polytétrafluoroéthylène, c'est à dire opaque et de couleur blanche. La densité a chuté de 938 kg/m³ à environ 600 kg/m³. On constate que l'échantillon modifié est beaucoup plus homogène qu'initialement.

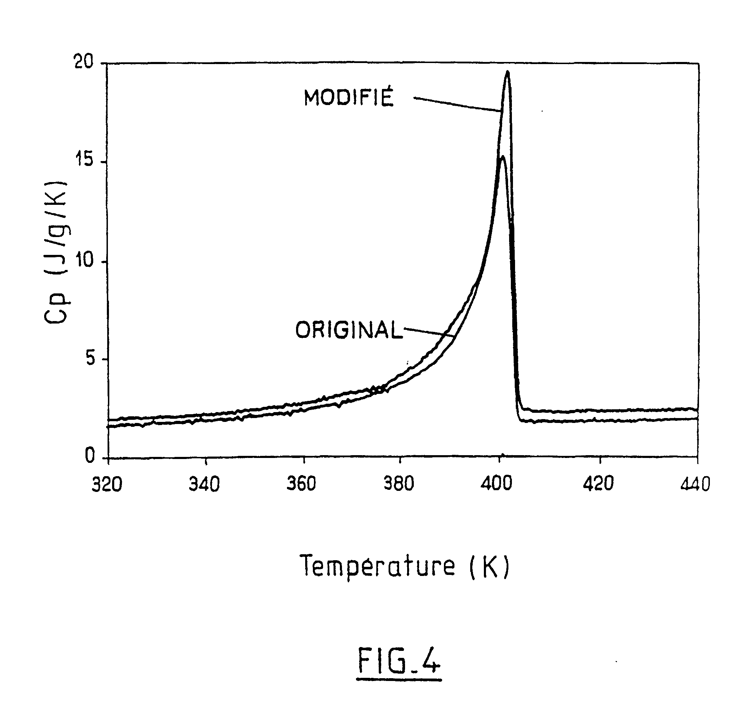

La figure 4 montre que les courbes de capacité thermique du MDPE modifié ont des transitions plus aiguës que celles du MDPE d'origine.

Les propriétés modifiées sont stables et ne changent pas à pression et température ambiantes, mais les modifications disparaissent sous l'effet d'une fusion et recristallisation à la pression atmosphérique.

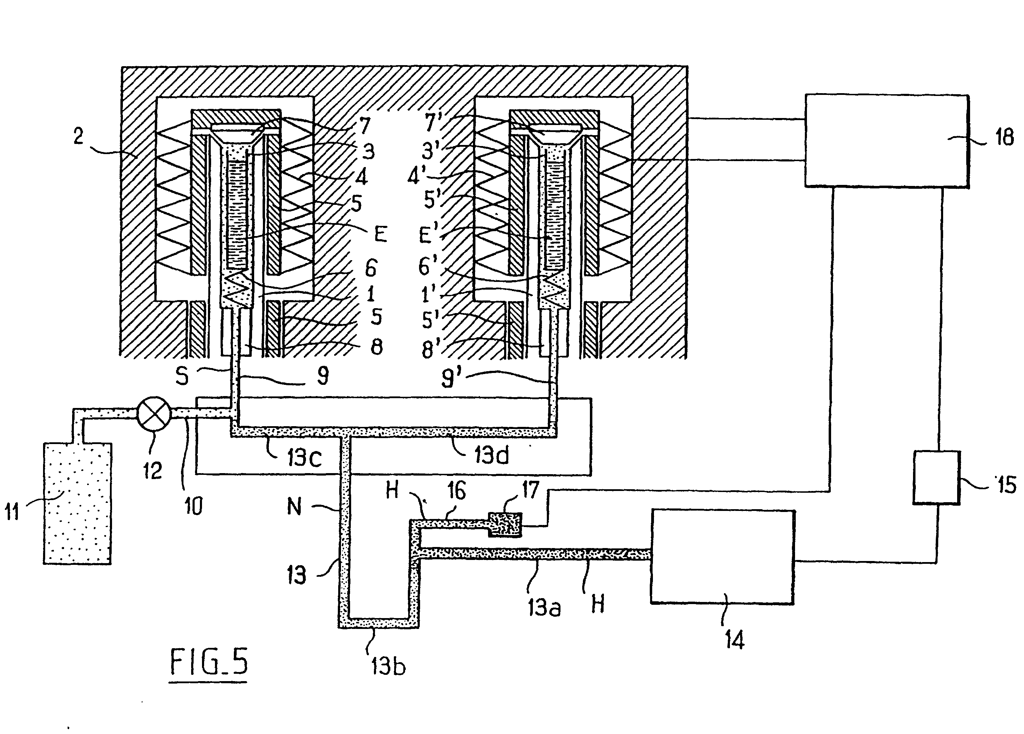

Le dispositif de la figure 1 est susceptible d'être modifié. La figure 5 montre par exemple une variante de dispositif dans laquelle deux cellules 1, 1' sont prévues, respectivement pour recevoir l'échantillon à étudier et le fluide supercritique et pour recevoir un échantillon et le fluide neutre par une canalisation 13d.

Les mêmes références, avec ou sans exposant prime, désignent des moyens correspondants sur les figures 1 et 5.

On décrira ci-après le fonctionnement du dispositif de la figure 5 en prenant comme exemple les modifications de la morphologie d'un polyéthylène semicristallin par augmentation et abaissement linéaires de la température sous la pression constante contrôlée du méthane supercritique ou par abaissement et augmentation linéaires de la pression du méthane supercritique à température constante.

On introduit un échantillon de la substance à modifier, dans l'exemple d'application - du polyéthylène de moyenne densité-, dans l'ampoule de la cellule 1, la cellule de référence 1' étant cette fois isolée du système hydraulique et servant seulement de témoin thermique connu.

On lave l'échantillon sous écoulement du méthane à température ambiante pour purger les impuretés. Ensuite, on ferme la cellule expérimentale, on l'introduit dans le détecteur calorimétrique se trouvant dans le thermostat et on introduit et on comprime le fluide jusqu'à la pression initiale nécessaire pour faire travailler la pompe haute pression 15.

On procède ensuite selon le mode opératoire choisi: isobare ou isotherme.

Dans le cas du mode isobare réalisé avec le concours du contrôleur logique 18 on comprime le système étudié par la pompe haute pression 14 jusqu'à la pression désirée. Après établissement de l'équilibre thermique et mécanique, la température est programmée avec une très faible vitesse constante, la pression étant maintenue constante, et on enregistre le thermogramme calorimétrique ainsi que le nombre de pas du moteur utilisés pour compenser le changement du volume pendant l'échauffement. Après la fin du changement de phase, observé surtout sur le thermogramme calorimétrique, on arrête l'échauffement et on commence le refroidissement avec la même vitesse de programmation de la température.

Les premiers thermogrammes aussi bien de fusion que de solidification contiennent les effets thermiques de changements de phase et les effets de l'interaction du gaz supercritique avec la substance modifiée. On continue donc les procédures d'échauffement et de refroidissement jusqu'à ce que les thermogrammes aient la forme stable. Cette forme stable peut être d'ailleurs facilement comparée avec la forme des thermogrammes respectifs obtenus pour la même substance avec les mêmes procédures, mais avec le gaz supercritique remplacé par un fluide neutre, par exemple du mercure. On peut réaliser ce remplacement en fermant la vanne 12 et en plaçant un échantillon vierge dans la cellule 1 ; le fluide hydraulique neutre (mercure) est ensuite amené vers le haut jusqu'à l'extrémité supérieure de cette cellule à l'aide de la pompe 14, après quoi la cellule est fermée avec le polymère plongé dans le mercure.

Il est tout aussi possible de réaliser cette comparaison dans une seule expérience où la cellule expérimentale 1 aussi bien que la cellule de référence 1' contiennent les échantillons du même polymère, mais la cellule 1 étant comprimée avec du gaz supercritique et la cellule de référence, 1' comprimée avec du mercure.

Dans le cas du mode isotherme réalisé avec le concours du contrôleur logique 18 on comprime le système étudié par la pompe haute pression jusqu'à la plus haute pression désirée et on élève la température jusqu'à la valeur choisie. Après avoir atteint l'équilibre thermique et mécanique, on réduit la pression avec une très faible vitesse constante, la température étant maintenue constante, et on enregistre le thermogramme calorimétrique ainsi que le nombre de pas du moteur utilisés pour compenser le changement du volume au cours de la décompression programmée. Après la fin du changement de phase, observé surtout sur le thermogramme calorimétrique, on arrête la décompression et on commence la compression avec la même vitesse de programmation de la pression au moins jusqu'à la fin de la solidification isotherme.

Les premiers thermogrammes aussi bien de fusion que de solidification contiennent les effets thermiques de changements de phase et les effets de l'interaction du gaz supercritique avec la substance modifiée. On continue donc les procédures de décompression et de compression jusqu'à ce que les thermogrammes aient la forme stable. Cette forme stable peut être d'ailleurs facilement comparée avec la forme de thermogrammes respectifs obtenus pour la même substance avec les mêmes procédures, mais avec le gaz supercritique remplacé par un fluide neutre, p.ex. du mercure ; ce remplacement est réalisé de la même façon que dans le mode isobare décrit au-dessus.

L'analyse détaillée de résultats obtenus fournit les paramètres thermodynamiques des changements de phase étudiés: pressions, entropies et volumes, aussi bien pour la substance étudiée vierge sous la pression d'un fluide neutre tel que le mercure que pour la substance modifiée sous la pression du gaz supercritique. La réalisation de telles comparaisons aussi bien dans les conditions isothermes que isobares n'est pas possible avec les procédés et les dispositifs actuellement connus.

Il va de soi que des modifications peuvent être apportées aux modes de réalisation qui viennent d'être décrits, notamment par substitution de moyens techniques équivalents, sans que l'on sorte pour cela de la portée de la présente invention.

## Revendications

1. Dispositif pour l'étude de l'effet d'un fluide supercritique sur la transition d'un matériau de l'une à l'autre des deux phases condensées, qui comprend :
- une cellule (1) apte à contenir un échantillon du matériau à étudier et à supporter les pressions et températures mises en jeu dans l'étude ;
- une source (11) de fluide supercritique (S) et une canalisation (9, 10) reliant cette source à l'intérieur de la cellule pour l'introduction contrôlée de ce fluide dans la cellule en sorte qu'il soit au contact de l'échantillon ;
- des moyens (2-5, 9-18) pour faire varier, de façon continue ou par paliers, selon un programme déterminé, la valeur d'un premier paramètre choisi parmi la pression (P) du fluide supercritique, la température (T) de la cellule et le volume (V) de l'échantillon dans la cellule, tout en maintenant un deuxième desdits paramètres à une valeur choisie, de façon à induire la transition ;
- des moyens (2-5, 9-18) pour enregistrer la variation du premier paramètre, la variation du troisième paramètre et les variations (ΔH) du flux thermique dans la cellule ;
- des moyens (18) pour effectuer des enregistrements analogues dans. des conditions similaires avec un fluide neutre au lieu du fluide supercritique.

2. Dispositif selon le revendication 1 et qui comprend une ampoule (3), éventuellement à paroi souple, pour contenir l'échantillon dans la cellule.

3. Dispositif selon l'une des revendications 1 et 2 et qui comprend une pompe haute pression (14) dont le piston est actionné par un moteur pas à pas (15), des moyens (13,N) pour transmettre la pression exercée par cette pompe au fluide supercritique, et des moyens (18) pour commander le moteur pas à pas et pour enregistrer les variations du nombre de pas du moteur.

4. Dispositif selon la revendication 3 et qui comprend un conduit (13a, 13b, 13c) reliant la sortie de pression de la pompe (14) à ladite canalisation (9) et contenant le fluide neutre (N).

5. Dispositif selon la revendication 5, dans lequel ledit conduit comporte une branche (13d) qui aboutit dans une autre cellule (1') identique à ladite cellule en sorte que le liquide neutre soit introduit dans cette autre cellule.

6. Dispositif selon la revendication 4 ou 5, dans lequel ledit conduit comporte à la sortie de la pompe une partie de conduit (13a) située en amont du fluide neutre contenu dans le conduit et qui contient un fluide hydraulique (H).

7. Dispositif selon l'une des revendications 1 à 6, dans lequel la cellule (1) ou chaque cellule (1, 1') est placée dans un détecteur calorimétrique (4 ; 4') entouré d'un thermostat (2).

8. Dispositif selon l'une des revendications 4 à 7 et qui comprend une centrale de commande et d'enregistrement (18) reliée au thermostat (2), aux détecteurs calorimétriques (4, 4'), au moteur pas à pas (15) et à un capteur de pression (17) qui reçoit la pression exercée par la pompe (14).

9. Procédé pour étudier l'effet d'un fluide supercritique sur la transition d'un matériau de l'une à l'autre de deux phases condensées, dans lequel :
- on introduit un échantillon du matériau et le fluide supercritique dans une cellule en sorte que le fluide soit au contact de l'échantillon ;
- on induit la transition en faisant varier, de façon continue ou par paliers, selon un programme déterminé, la valeur d'un premier paramètre choisi par la pression (P) du fluide, la température (T) de la cellule et le volume (V) de l'échantillon dans la cellule, tout en maintenant un deuxième desdits paramètres à une valeur choisie;
- on enregistre la variation du premier paramètre, la variation du troisième paramètre et la variation du flux thermique dans la cellule ;
- on recommence les opérations précédentes avec le même paramètre programmé et la même valeur choisie mais en utilisant un fluide neutre à la place du fluide supercritique, en mettant ou non le fluide neutre au contact de l'échantillon selon le cas ;
- et on compare les résultats obtenus avec les deux fluides pour évaluer l'effet du fluide supercritique sur les conditions de la transition.

10. Procédé selon la revendication 9, dans lequel on utilise un fluide supercritique choisi parmi le gaz carbonique, l'azote, le méthane, l'éthane, le propane ou leurs mélanges et les autres fluides capables d'être amenés à l'état supercritique.

11. Procédé selon l'une des revendications 9 et 10, dans lequel on utilise le mercure comme fluide neutre.

12. Procédé selon l'une des revendications 9 à 11, lequel on transmet au fluide supercritique la pression d'un fluide hydraulique actionné par un piston.

13. Procédé selon la revendication 11, dans lequel on réalise cette transmission au moyen du fluide neutre.

14. Procédé selon la revendication 13 dans lequel pour l'étude de la transition du matériau en présence du fluide neutre dans la cellule on utilise ledit fluide hydraulique pour pousser le fluide neutre jusque dans la cellule.

15. Procédé selon l'une des revendications 12 à 14, dans lequel on déplace ledit piston par l'action d'un moteur pas à pas pour créer ladite pression et on compte le nombre de pas du moteur nécessaire pour maintenir la pression à une valeur choisie de façon à déterminer la variation du volume de l'échantillon dans la cellule pendant la transition.

16. Procédé selon la revendication 9, dans lequel on réalise les étapes suivantes : a) introduction du fluide supercritique dans la cellule expérimentale sous la pression nécessaire pour faire travailler une pompe haute pression ; b) compression du fluide supercritique par la pompe haute pression jusqu'à la pression désirée ; c) initiation isobare de la fusion du matériau par augmentation contrôlée de la température et enregistrement simultané des changements du volume et du flux et de chaleur ; d) initiation isobare de la cristallisation isobare du matériau par abaissement contrôlé de la température et enregistrement simultané des changements du volume et du flux de chaleur.

17. Procédé selon la revendication 9, dans lequel on réalise les étapes suivantes: a) introduction du fluide supercritique dans la cellule expérimentale contenant le matériau sous la pression nécessaire pour faire travailler la pompe haute pression; b) compression du fluide supercritique par la pompe haute pression jusqu'à la pression désirée, c) initiation isotherme de la fusion de la substance par abaissement contrôlé de la pression et enregistrement simultané des changements du volume et du flux de chaleur; d) initiation isotherme de la cristallisation isotherme du matériau par augmentation contrôlée de la pression et enregistrement simultané des changements du volume et du flux de chaleur.

18. Procédé selon la revendication 9, dans lequel on modifie ledit matériau se trouvant à l'état fluide ou liquide en abaissant sa température sous la pression contrôlée du fluide supercritique.

19. Application d'un procédé selon l'une des revendications 9 à 18 au cas d'un matériau polymère pour déterminer quand la solution polymère / fluide supercritique est dans un état d'équilibre et quand cette solution devient saturée.

20. Application selon la revendication 19 dans laquelle on induit la transition du polymère en faisant varier la température à pression constante.

21. Application selon la revendication 20, dans laquelle dans les mêmes conditions de pression, on réalise un échauffement lent pour obtenir un système homogène polymère / gaz supercritique saturé puis un refroidissement lent pour faire condenser une phase micro-mousse ou nano-mousse, tout en enregistrant tous les paramètres (P,T, ΔH et ΔV) des transitions observées.

22. Application selon la revendication 19, dans laquelle on induit la transition du polymère en faisant varier la pression à température constante.

23. Application selon la revendication 22 dans laquelle, dans les mêmes conditions de pression, on réalise un échauffement lent pour obtenir un système homogène polymère /gaz supercritique saturé puis une compression lente pour faire condenser une phase micro-mousse ou nano-mousse, tout en enregistrant tous les paramètres (P,T, ΔH et ΔV) des transitions observées.

24. Application selon l'une des revendications 21 et 23 dans laquelle la forme stable du thermogramme, qui ne change pas au cours des transitions successives sous l'atmosphère du gaz supercritique, est l'indicateur de la fin de la formation de la nouvelle phase micro-mousse ou nano-mousse.

## Claims

1. Apparatus for studying the effect of a supercritical fluid on the transition of a material from one of two condensed phases to the other, which comprises:
- a cell (1) suitable for containing a specimen of the material to be studied and for withstanding the pressures and temperatures involved in the study;
- a source (11) of supercritical fluid (S) and a pipe (9, 10) connecting this source to the inside of the cell for controlled introduction of this fluid into the cell so that it is in contact with the specimen;
- means (2-5, 9-18) for varying, continuously or in steps, according to a defined program, the value of a first parameter chosen from the pressure (P) of the supercritical fluid, the temperature (T) of the cell and the volume (V) of the specimen in the cell, whilst keeping a second of the said parameters at a chosen value, so as to induce the transition;
- means (2-5, 9-18) for recording the variation in the first parameter, the variation in the third parameter and the variations (ΔH) in the heat flux in the cell;
- means (18) for making analogue recordings under similar conditions with a neutral fluid instead of the supercritical fluid.

2. Apparatus according to Claim 1, which comprises an ampoule (3), optionally with a flexible wall, for containing the specimen in the cell.

3. Apparatus according to either of Claims 1 and 2, which comprises a high-pressure pump (14) whose piston is actuated by a stepper motor (15), means (13, N) for transmitting the pressure exerted by this pump to the supercritical fluid, and means (18) for controlling the stepper motor and for recording the variations in the number of steps of the motor.

4. Apparatus according to Claim 3, which comprises a duct (13a, 13b, 13c) connecting the pressure outlet of the pump (14) to the said pipe (9) and containing the neutral fluid (N).

5. Apparatus according to Claim 5, in which the said duct includes a branch (13d) which terminates in another cell (1') identical to the said cell so that the neutral liquid is introduced into this other cell.

6. Apparatus according to Claim 4 or 5, in which the said duct includes, at the outlet of the pump, a duct part (13a) which lies upstream of the neutral fluid contained in the duct and contains a hydraulic fluid (H).

7. Apparatus according to one of Claims 1 to 6, in which the cell (1) or each cell (1, 1') is placed in a calorimetric detector (4; 4') surrounded by a thermostat (2).

8. Apparatus according to one of Claims 4 to 7, which comprises a control and recording unit (18) connected to the thermostat (2), to the calorimetric detectors (4, 4'), to the stepper motor (15) and to a pressure sensor (17) which receives the pressure exerted by the pump (14).

9. Method for studying the effect of a supercritical fluid on the transition of a material from one of two condensed phases to the other, in which:
- a specimen of the material and the supercritical fluid are introduced into a cell so that the fluid is in contact with the specimen;
- the transition is induced by varying, continuously or in steps, according to a defined program, the value of a first parameter chosen by the pressure (P) of the fluid, the temperature (T) of the cell and the volume (V) of the specimen in the cell, whilst keeping a second of the said parameters at a chosen value;
- the variation in the first parameter, the variation in the third parameter and the variation in the heat flux in the cell are recorded;
- the above operations are repeated with the same program parameter and the same chosen value but using a neutral fluid instead of the supercritical fluid, by bringing or not bringing the neutral fluid into contact with the specimen depending on the case;
- and the results obtained for the two fluids are compared in order to evaluate the effect of the supercritical fluid on the transition conditions.

10. Method according to Claim 9, in which the supercritical fluid used is chosen from carbon dioxide, nitrogen, methane, ethane, propane, or mixtures thereof, and the other fluids capable of being brought to the supercritical state.

11. Method according to one of Claims 9 and 10, in which mercury is used as the neutral fluid.

12. Method according to one of Claims 9 to 11, in which the pressure of a hydraulic fluid actuated by a piston is transmitted to the supercritical fluid.

13. Method according to Claim 11, in which this transmission is carried out by means of the neutral fluid.

14. Method according to Claim 13, in which, for studying the transition of the material in the presence of the neutral fluid in the cell, the said hydraulic fluid is used to push the neutral fluid right into the cell.

15. Method according to one of Claims 12 to 14, in which the said piston is displaced by the action of a stepper motor in order to create the said pressure and the number of steps of the motor needed to keep the pressure at a chosen value is counted so as to determine the variation in the volume of the specimen in the cell during the transition.

16. Method according to Claim 9, in which the following steps are carried out: a) introduction of the supercritical fluid into the experimental cell at the pressure needed to make a high-pressure pump work; b) compression of the supercritical fluid by the high-pressure pump to the desired pressure; c) isobaric initiation of the melting of the material by controlled increase in the temperature and simultaneous recording of the changes in the volume and in the heat flux; d) isobaric initiation of the isobaric crystallization of the material by controlled reduction in the temperature and simultaneous recording of the changes in the volume and in the heat flux.

17. Method according to Claim 9, in which the following steps are carried out: a) introduction of the supercritical fluid into the experimental cell containing the material at the pressure needed to make the high-pressure pump work; b) compression of the supercritical fluid by the high-pressure pump to the desired pressure; c) isothermal initiation of the melting of the substance by controlled reduction in the pressure and simultaneous recording of the changes in the volume and in the heat flux; d) isothermal initiation of the isothermal crystallization of the material by controlled increase in the pressure and simultaneous recording of the changes in the volume and in the heat flux.

18. Method according to Claim 9, in which the said material in the fluid or liquid state is modified by reducing its temperature below the controlled pressure of the supercritical fluid.

19. Application of a method according to one of Claims 9 to 18 to the case of a polymer material for determining when the supercritical fluid/polymer solution is in an equilibrium state and when this solution becomes saturated.

20. Application according to Claim 19, in which the transition of the polymer is induced by varying the temperature at constant pressure.

21. Application according to Claim 20, in which under the same pressure conditions, a slow heating is effected in order to obtain a homogeneous, saturated supercritical gas/polymer system followed by a slow cooling in order to make a microfoam or nanofoam phase condense, whilst recording all the parameters (P,T, ΔH and ΔV) of the observed transitions.

22. Application according to Claim 19, in which the transition of the polymer is induced by varying the pressure at constant temperature.

23. Application according to Claim 22, in which, under the same pressure conditions, a slow heating is effected in order to obtain a homogeneous, saturated supercritical gas/polymer system followed by a slow compression in order to make a microfoam or nanofoam phase condense, whilst recording all the parameters (P,T, ΔH and ΔV) of the observed transitions.

24. Application according to either of Claims 21 and 23, in which the stable shape of the thermogram, which does not change during the successive transitions under the atmosphere of the supercritical gas, is the indicator of the end of formation of the new microfoam or nanofoam phase.

## Patentansprüche

1. Vorrichtung zum Untersuchen der Wirkung eines superkritischen Fluides auf den Übergang eines Materials von einer Phase zur anderen von zwei Kondensationsphasen, die folgendes umfaßt:
- eine Zelle (1), die geeignet ist, eine Probe des zu untersuchenden Materials aufzunehmen und die für die Untersuchung angewandten Drücke und Temperaturen auszuhalten;
- eine Quelle (11) eines superkritischen Fluides (S) und ein Leitungssystem (9, 10), das diese Quelle mit dem Inneren der Zelle zum gesteuerten Einführen dieses Fluides in die Zelle verbindet, damit dieses in Kontakt mit der Probe ist;
- Mittel (2-5, 9-18) zum kontinuierlichen oder stufenweisen Variieren entsprechend einem vorbestimmten Programm des Wertes eines ersten Parameters, der aus dem Druck (P) des superkritischen Fluides, der Temperatur (T) der Zelle und dem Volumen (V) der Probe in der Zelle ausgewählt ist, wobei ein zweiter dieser Parameter dabei auf einem ausgewählten Wert gehalten wird, damit der Übergang bewirkt wird;
- Mittel (2-5, 9-18) zum Aufnehmen der Variation des ersten Parameters, der Variation des dritten Parameters und der Variationen (ΔH) des Wärmeflusses in der Zelle;
- Mittel (18) zum Durchführen analoger Aufnahmen unter ähnlichen Bedingungen mit einem neutralen Fluid anstelle des superkritischen Fluides.

2. Vorrichtung nach Anspruch 1, die eine Ampulle (3), ggf. mit einer weichen Wandung, zum Aufnehmen der Probe in der Zelle umfaßt.

3. Vorrichtung nach einem der Ansprüche 1 und 2, die eine Hochdruckpumpe (14), deren Kolben von einem Schrittmotor (15) angetrieben wird, Mittel (13,N) zum Übertragen des von dieser Pumpe ausgeübten Druckes auf das superkritische Fluid, und Mittel (18) zum Steuern des Schrittmotors und zum Aufnehmen der Variationen der Schrittzahl des Motors umfaßt.

4. Vorrichtung nach Anspruch 3, die eine Leitung (13a, 13b, 13c) umfaßt, die den Druckausgang der Pumpe (14) mit dem Leitungssystem (9) verbindet und das neutrale Fluid (N) enthält.

5. Vorrichtung nach Anspruch 5, bei der die Leitung eine Verzweigung (13d) umfaßt, die in eine andere Zelle (1') mündet, die identisch zu der besagten Zelle ist, damit das neutrale Fluid in diese andere Zelle eingeführt wird.

6. Vorrichtung nach Anspruch 4 oder 5, bei der die besagte Leitung am Ausgang der Pumpe einen Leitungsabschnitt (13a) umfaßt, der stromauf des in der Leitung enthaltenen neutralen Fluides liegt und der ein Hydraulikfluid (H) enthält.

7. Vorrichtung nach einem der Ansprüche 1 bis 6, bei der die Zelle (1) oder jede Zelle (1, 1') in einem kalorimetrischen Detektor (4; 4') angeordnet ist, der von einem Thermostat (2) umgeben ist.

8. Vorrichtung nach einem der Ansprüche 4 bis 7, die eine Steuerund Aufnahmezentrale (18) umfaßt, die mit dem Thermostat (2), den kalorimetrischen Detektoren (4,4'), dem Schrittmotor (15) und einem Druckmesser (17) verbunden ist, der den von der Pumpe (14) ausgeübten Druck empfängt.

9. Verfahren zum Untersuchen der Wirkung eines superkritischen Fluides auf den Übergang eines Materials von einer Phase zur anderen zweier kondensierter Phasen, bei dem:
- eine Probe des Materials und das superkritische Fluid in eine Zelle eingeführt werden, damit das Fluid in Kontakt mit der Probe ist;
- der Übergang bewirkt wird, indem gemäß einem vorbestimmten Programm der Wert eines ersten Parameters kontinuierlich oder stufenweise variiert wird, der aus dem Druck (P) des Fluides, der Temperatur (T) der Zelle und dem Volumen (V) der Probe in der Zelle ausgewählt ist, wobei ein zweiter der besagten Parameter auf einem ausgewählten Wert gehalten wird;
- die Variation des ersten Parameters, die Variation des dritten Parameters und die Variation des Wärmeflusses in der Zelle aufgenommen werden;
- die vorgenannten Operationen mit dem gleichen programmierten Parameter und dem gleichen ausgewählten Wert erneut begonnen werden, indem jedoch ein neutrales Fluid anstelle des superkritischen Fluides verwendet wird, indem vom Fall abhängig das neutrale Fluid in Kontakt oder nicht in Kontakt mit der Probe gebracht wird;
- und die mit den beiden Flüssigkeiten erhaltenen Ergebnisse verglichen werden, um die Wirkung des superkritischen Fluides auf die Bedingungen des Überganges zu bewerten.

10. Verfahren nach Anspruch 9, bei dem ein superkritisches Fluid verwendet wird, das aus dem Kohlendioxid-, dem Stickstoff-, dem Methan-, dem Ethan-, dem Propangas oder deren Mischungen und den anderen Fluiden ausgewählt ist, die geeignet sind, in den superkritischen Zustand gebracht zu werden.

11. Verfahren nach einem der Ansprüche 9 und 10, bei dem Quecksilber als neutrales Fluid verwendet wird.

12. Verfahren nach einem der Ansprüche 9 bis 11, bei dem dem superkritischen Fluid der Druck eines durch einen Kolben angetriebenen Hydraulikfluides übertragen wird.

13. Verfahren nach Anspruch 11, bei dem diese Übertragung mittels des neutralen Fluides verwirklicht wird.

14. Verfahren nach Anspruch 13, bei dem zur Untersuchung des Übergangs des Materials bei Vorliegen des neutralen Fluides in der Zelle das Hydraulikfluid verwendet wird, um das neutrale Fluid bis in die Zelle zu drücken.

15. Verfahren nach einem der Ansprüche 12 bis 14, bei dem der Kolben durch Antrieb eines Schrittmotors verschoben wird, um den Druck zu erzeugen, und bei dem die Schrittzahl des Motors gezählt wird, die erforderlich ist, um den Druck auf einem ausgewählten Wert zu halten, um die Variation des Volumens der Probe in der Zelle während des Übergangs zu bestimmen.

16. Verfahren nach Anspruch 9, bei dem die folgenden Schritte durchgeführt werden: a) Einführen des superkritischen Fluides in die Experimentierzelle unter dem erforderlichen Druck, um eine Hochdruckpumpe arbeiten zu lassen; b) Komprimierung des superkritischen Fluides durch die Hochdruckpumpe bis zum gewünschten Druck; c) Isobare Einleitung des Schmelzens des Materials durch gesteuerte Temperaturerhöhung und gleichzeitiges Aufnehmen der Änderung des Volumens und des Wärmeflusses; d) Isobare Einleitung der isobaren Kristallisierung des Materials durch gesteuerte Temperatursenkung und gleichzeitiges Aufnehmen der Änderungen des Volumens und des Wärmeflusses.

17. Verfahren nach Anspruch 9, bei dem die folgenden Schritte durchgeführt werden: a) Einführen des superkritischen Fluides in die das Material enthaltende Experimentierzelle unter dem erforderlichen Druck, um die Hochdruckpumpe arbeiten zu lassen; b) Komprimieren des superkritischen Fluides mittels der Hochdruckpumpe bis zum gewünschten Druck; c) Isotherme Einleitung des Schmelzens der Substanz durch gesteuerte Drucksenkung und gleichzeitiges Aufnehmen der Änderungen des Volumens und des Wärmeflusses; d) Isotherme Einleitung der isothermen Kristallisierung des Materials durch gesteuerte Druckerhöhung und gleichzeitiges Aufnehmen der Änderungen des Volumens und des Wärmeflusses.

18. Verfahren nach Anspruch 9, bei dem das sich im fluiden oder flüssigen Zustand befindliche Material verändert wird, indem seine Temperatur unter dem gesteuerten Druck des superkritischen Fluides gesenkt wird.

19. Anwendung eines Verfahrens nach einem der Ansprüche 9 bis 18 im Falle eines polymeren Materials, zum Bestimmen, wann sich die Lösung aus Polymer/superkritischem Fluid in einem Gleichgewichtszustand befindet, und wann diese Lösung gesättigt wird.

20. Anwendung nach Anspruch 19, bei der der Übergang des Polymers durch Variieren der Temperatur bei konstantem Druck bewirkt wird.

21. Anwendung nach Anspruch 20, bei der unter gleichen Druckbedingungen eine langsame Erwärmung durchgeführt wird, um ein homogenes System aus Polymer/gesättigtem superkritischen Gas zu erhalten, anschließend eine langsame Abkühlung, um eine Mikroschaumoder Nanoschaumphase kondensieren zu lassen, wobei dabei all die Parameter (P,T, ΔH und ΔV) der überwachten Übergänge aufgenommen werden.

22. Anwendung nach Anspruch 19, bei der der Übergang des Polymers bewirkt wird, indem der Druck bei konstanter Temperatur variiert wird.

23. Anwendung nach Anspruch 22, bei der unter gleichen Druckbedingungen eine langsame Erwärmung durchgeführt wird, um ein homogenes System aus Polymer/gesättigtem superkritischen Gas zu erhalten, anschließend eine langsame Kompression, um eine Mikroschaumoder Nanoschaumphase kondensieren zu lassen, wobei dabei all die Parameter (P, T,. ΔH und ΔV) der beobachteten Übergänge aufgenommen werden.

24. Verwendung nach einem der Ansprüche 21 bis 23, bei der die stabile Form des Temperaturbildes, die sich während aufeinanderfolgender Übergänge in der Umgebung des superkritischen Gases nicht ändert, die Anzeige des Endes der Bildung der neuen Mikroschaum- oder Nanoschaumphase ist.
